(19) Europäisches Patentamt

European Patent Office

Office européen des brevets

(11) **EP 0 528 891 B1**

(12) **EUROPEAN PATENT SPECIFICATION**

(45) Date of publication and mention
of the grant of the patent:
**05.07.2000 Bulletin 2000/27**

(51) Int. Cl.[7]: **A61K 38/08**

(86) International application number:
**PCT/US91/02772**

(21) Application number: **91909190.0**

(22) Date of filing: **23.04.1991**

(87) International publication number:
**WO 91/16355 (31.10.1991 Gazette 1991/25)**

(54) **METHOD FOR INCREASING BLOOD-BRAIN BARRIER PERMEABILITY**

VERFAHREN ZUR STEIGERUNG DER BLUT-HIRN-SCHRANKEN-DURCHLÄSSIGKEIT

PROCEDE SERVANT A AUGMENTER LA PERMEABILITE DE LA BARRIERE HEMATO-
ENCEPHALIQUE

(84) Designated Contracting States:
**AT BE CH DE DK ES FR GB GR IT LI LU NL SE**

(30) Priority: **23.04.1990 US 512913**

(43) Date of publication of application:
**03.03.1993 Bulletin 1993/09**

(73) Proprietor: **ALKERMES, INC.
Cambridge, MA 02139-4136 (US)**

(72) Inventors:
• **MALFROY-CAMINE, Bernard
Arlington, MA 02174 (US)**

• **SMART, Janet, L.
Londonderry, NH 03053 (US)**

(74) Representative:
**Kirkham, Nicholas Andrew et al
Graham Watt & Co.,
Riverhead
Sevenoaks, Kent TN13 2BN (GB)**

(56) References cited:
**FR-M- 7 979**

• **TIBTECH 12 (june 1994) 239**

## Description

Background

As our understanding of the nervous system and its related disorders increases, a wider range of therapeutic and diagnostic agents will become available. Once these agents have been identified, it will be necessary to deliver them to sites of diseased tissue in the central nervous system. Unfortunately, the existence of the blood-brain barrier limits the free passage of many types of molecules from the blood to cells of the central nervous system.

The physiological basis for the blood-brain barrier is the brain capillaries, which are comprised of endothelial cells (Goldstein, et al. , Scientific American, 255: 74-83 (1986); Pardridge, W.M., Endocrin. Rev., 7: 314-330 (1986)). These endothelial cells are different from those found in other tissues of the body. In particular, they form complex tight junctions between themselves. The actual blood-brain barrier is formed by these high-resistance tight intercellular junctions which form a continuous wall against the passive movement of many molecules from the blood to the brain. These cells are also different in that they have few pinocytotic vesicles, which in other tissues allow somewhat unselective transport across the capillary wall. In addition, continuous gaps or channels running through the cells, which would allow unrestrained passage, are absent.

One function of the blood-brain barrier is to protect the brain from fluctuations in blood chemistry. However, this isolation of the brain from the bloodstream is not complete. There does exist an exchange of nutrients and waste products. The presence of specific transport systems within the capillary endothelial cells assures that the brain receives, in a controlled manner, all of the compounds required for normal growth and function. The obstacle presented by the blood-brain barrier is that, in the process of protecting the brain, it excludes many potentially useful therapeutic and diagnostic agents.

There are several techniques that either physically break through the blood-brain barrier or circumvent it to deliver therapeutic or diagnostic agents. Among these are intrathecal injections, surgical implants, and osmotic techniques.

Intrathecal injection allows administration of agents directly into the brain ventricles and spinal fluid by puncturing the membranes surrounding the brain. Sustained delivery of agents directly into the spinal fluid can be attained by the use of infusion pumps that are implanted surgically. These spinal fluid delivery techniques are used to treat brain cancers, infections, inflammation and pain. However, they often do not result in deep penetration of substances into the brain.

Clinicians prefer to avoid intrathecal injections because they frequently are ineffective and can be dangerous. Substances injected intrathecally are distributed unevenly, slowly and incompletely in the brain. Since the volume of the spinal fluid is small, increases in intracerebral pressure can occur with repeated injections. Furthermore, improper needle or catheter placement can result in seizure, bleeding, encephalitis and a variety of other severe side effects.

An osmotic approach has been used by Dr. Edward Neuwelt at the University of Oregon to deliver chemotherapeutics and imaging antibodies to tumors in the brain. (Neuwelt, E.A., Implication of the Blood-Brain Barrier and its Manipulation, Vols 1 & 2, Plenum Press, N.Y. (1989)) This technique involves an arterial injection of a bolus of a hypertonic mannitol solution. The osmotic differential exerted by the mannitol causes the endothelial cells forming the barrier to shrink, opening gaps between them for a brief period. During this period, the drug is administered into the arterial system and is carried directly into the brain. The osmotic approach demonstrates that once past the barrier, therapeutic agents can be effectively distributed throughout the brain.

Because of the many risks involved, a 24- to 48-hour period in an intensive care unit is necessary following osmotic treatment. Mannitol can cause permanent damage (including blindness) to the eye. If the barrier is permeable for too long, brain edema results. Cells of the brain also can be damaged when neurotoxic substances in the blood, not generally accessible to the brain, are able to cross the barrier. Finally, there is a significant incidence of seizures in patients during and after the procedure.

Summary of the Invention

The present invention pertains to a method of increasing the permeability of the blood-brain barrier of a patient to a therapeutic or diagnostic imaging agent contained in the patient's bloodstream. This method comprises intravenous co-administration to the patient of an effective amount of a bradykinin agonist of blood-brain barrier permeability. The agent to be delivered to the brain can be either an endogenous molecule or an exogenous molecule that is co-administered sequentially or simultaneously with the bradykinin agonist of blood-brain barrier permeability.

An advantage of the present invention is that it provides a practical means of increasing the permeability of the blood-brain barrier by the intravenous administration of a bradykinin agonist of blood-brain barrier permeability while co-administering a molecule of therapeutic, prophylactic or diagnostic value. In contrast to osmotic treatment or intrathecal delivery, intravenous injection of a bradykinin agonist of blood-brain barrier permeability is significantly less traumatic, does not require surgery and is unlikely to necessitate anesthesia. Furthermore, in contrast to the osmotic techniques which allow entry into the brain of a large variety of molecules present in the blood, including protein, the bradykinin agonist of blood-brain barrier permeability preferentially induces passage through the blood-brain barrier of small molecules.

The intravenous route of administration of a bradykinin agonist of blood-brain barrier permeability offers a number of significant advantages over other routes of administration (subcutaneous or intramuscular injection, as well as the more drastic measures of intrathecal or carotid artery injection). For example, the intravenous route of administration affords a more convenient method for providing rapid drug action. The intravenous route also offers better control over the rate of administration of drugs; prolonged action can be provided by administering an infusion either intermittently or over a prolonged period of time. Also, slow intravenous administration of a drug permits termination of the infusion if sensitivity occurs. In addition, certain drugs, because of their irritating properties, cause pain and trauma when given by the intramuscular or subcutaneous route and must be given intravenously. Additionally, some drugs cannot be absorbed by any other route or are unstable in the presence of gastric juices. The intravenous route also affords a means of administration for the patient who cannot tolerate fluids and drugs by the gastrointestinal route. Delivering therapeutic and diagnostic agents into the brain by an intravenous technique is unique and will improve dramatically and immediately the clinician's ability to understand, diagnose and treat diseases of the central nervous system.

Brief Description of the Drawings

Figure 1 is a graphic representation of brain uptake ($\mu$l/g) of sucrose and bovine serum albumin (BSA) coadministered with bradykinin at several doses of bradykinin ($\mu$g/mouse).

Figure 2 is a graphic representation of the time course of brain uptake of sucrose after administration of a specific amount (10 $\mu$g) of a bradykinin analogue into different animals.

Figure 3 is a graphic representation of the time course of brain uptake of sucrose after the administration of a specific amount (100 $\mu$g) of a bradykinin analogue.

Figure 4a is a graphic representation of brain uptake of sucrose, displayed as percent of injected dose, following the administration of bradykinin and captopril or bradykinin analogues.

Figure 4b is a graphic representation of brain uptake of sucrose, displayed as percent of maximum uptake, following the administration of bradykinin and captopril or bradykinin analogues.

Figure 5 is a histogram which illustrates the effect of co-administered bradykinin on the antinociceptive activity of loperamide in the mouse tail flick assay.

Figure 6 is a histogram which illustrates the effect of a co-administered bradykinin analogue on the antinociceptive activity of loperamide in the mouse tail flick assay.

Figure 7 is a graphic representation of the effects of a dopamine analogue, diacetyldopamine (AcDA), on the motor activity of rats when administered alone, with bradykinin or with bradykinin and haloperidol (Haldol).

Figure 8 is a graphic representation of the effects of no treatment (control), treatment with cisplatin, treatment with captopril and co-administered bradykinin, and treatment with cisplatin, captopril and coadministered bradykinin in the survival time (days) of rats implanted with a brain tumor, with treatments occuring at days 4 and 6 after tumor implantation.

Figure 9 is a graphic representation of the effects of no treatment, treatment with cisplatin and treatment with cisplatin, capropril and co-administered bradykinin on survival time (days) of rats implanted with a brain tumor, with treatment period lasting from day 4 to day 14.

Figure 10 is a graphic representation of the effects of no treatment; treatment with cisplatin; treatment with bradykinin, captopril and cisplatin; and treatment with a bradykinin analogue and cisplatin on survival time (days) of rats implanted with a brain tumor.

Figure 11 is a graphic representation of the brain uptake of an imaging agent intravenously injected 3 minutes after either saline or one of three different amounts of bradykinin analogue was intravenously injected into rats.

Figure 12 is a histogram which illustrates the effect of a bradykinin analogue co-administered with an imaging agent on the uptake of the imaging agent into the brain at various times after injection.

Figure 13 is a graphic representation of the effects of a dopamine receptor antagonist (domperidone) co-administered with a bradykinin analogue on apomorphine-induced motor activity of rats.

Figure 14 is a graphic representation of the effects of an angiotensin II analogue co-administered with a bradykinin analogue on drinking behavior in rats.

Figure 15 is a histogram which illustrates the effects of an angiotensin II analogue and inhibitor co-administered with a bradykinin analogue on drinking behavior in rats.

Detailed Description of the Invention

This invention relates to a method for increasing the permeability of the blood-brain barrier of a patient to a therapeutic or diagnostic imaging agent present in the patient's bloodstream. The increase in permeability of the blood-brain barrier is accomplished by intravenously co-administering a bradykinin agonist of blood-brain barrier permeability to the patient. By the phrase "bradykinin agonist of blood-brain barrier permeability" is meant a compound that elicits an increase in blood-brain barrier permeability in the manger of bradykinin.

The patient can be any animal which possesses a central nervous system (i.e., a brain). Examples of patients include mammals, such as humans, domestic animals (e.g, dog, cat, cow or horse) and animals intended for experimental purposes (e.g., mice, rats, rabbits).

The agent in the patient's bloodstream can be exogenous to the patient. For example, it can be a neuropharmaceutical agent which has a therapeutic or prophylactic effect on a neurological disorder. Examples of neurological disorders include cancer (e.g., brain tumors), Autoimmune Deficiency Syndrome (AIDS), epilepsy, Parkinson's disease, multiple sclerosis, neurodegenerative disease, trauma, depression, Alzheimer's disease, migraine, pain, or a seizure disorder.

Classes of neuropharmaceutical agents which can be used in this invention include antimicrobial agents, antiparasitic agents, agents that act on the autonomic nervous system including adrenergic agents and catecholaminergic agents, anticonvulsants, nucleotide analogues, antineoplastic agents, anti-trauma agents, excitatory amino acids and inhibitors and other classes of agents used to treat or prevent a neurological disorder. Examples of antibiotics include amphotericin B, gentamicin sulfate, pyrimethamine and penicillin. An example of an adrenergic agent (including blockers) is atenolol. Examples of catecholaminergic agents include dopamine, diacetyldopamine and domperidone. Examples of antineoplastic agents include adriamycin, methotrexate, cyclophosphamide, etoposide, carboplatin and cisplatin. An example of an anticonvulsant which can be used is valproate. Examples of anti-trauma agents which can be used include calpain inhibitors, channel blockers, glutamate chelators and oxygen radical scavengers. Nucleotide analogues which can be used include azido thymidine (AZT), dideoxy Inosine (ddI) and dideoxy Cytidine (ddC).

The agent in the patient's bloodstream can also be diagnostic imaging or contrast agents. Examples of diagnostic agents include substances that are labelled with radioactivity, such as 99m-Tc glucoheptonate or substances used in Magnetic Resonance Imaging (MRI) procedures, such as Gadolinium-doped chelation agents (e.g., Gd-DTPA).

The route of administration of exogenous agents to the patient's bloodstream can be parenterally by subcutaneous, intravenous or intramuscular injection or by absorption through a bodily tissue, such as the digestive tract, the respigatory system or the skin. The form in which the agent is administered (e.g., capsule, tablet, solution, emulsion) will depend, at least in part, on the route by which it is administered.

The administration of the exogenous agent to the patient's bloodstream and the intravenous injection of bradykinin agonist of blood-brain barrier permeability can occur simultaneously or sequentially in time. For example, a therapeutic drug can be administered orally in tablet form while the intravenous administration of a bradykinin agonist of blood-brain barrier permeability is given later (e.g. 30 minutes). This is to allow time for the drug to be absorbed in the gastrointestinal tract and taken up by the bloodstream before the agonist is given to increase the permeability of the blood-brain barrier to the drug. On the other hand, the bradykinin agonist of blood-brain barrier permeability can be administered before or at the same time as an intravenous injection of a drug. Thus, the term "co-administration" is used herein to mean that the bradykinin agonist of blood-brain barrier permeability and the exogenous molecule will be administered at times that will achieve significant concentrations in the blood producing the simultaneous effects of increasing the permeability of the blood-brain barrier and allowing the maximum passage of the exogenous agent from the blood to the interstitial fluid of the central nervous system.

In addition, the agent to be delivered to the brain, via the patient's bloodstream can be endogenous to the patient. That is, it can be a biological product that is naturally synthesized and produced by the patient. Examples of such biological products include sugars, such as glucose, and small peptides, such as enkephalins and thyroid stimulating hormone releasing factor.

Compounds are termed agonists when they increase or elicit a physiological response by interacting with structural moieties at particular anatomical regions of the body. For purposes of this invention, a compound is an agonist of blood-brain barrier permeability when it interacts with structural moieties anatomically associated with the blood-brain barrier to significantly increase the permeability of the blood-brain barrier for the agent of interest. This effect of increased permeability of the blood-brain barrier is believed to operate through a tissue-associated receptor mediated event, probably through the $B_2$ receptors. Other tissue-associated receptors may similarly be involved in this process to increase the permeability of the blood-brain barrier, particularly when peptides or compounds other than bradykinin or bradykinin analogues are utilized as the agonist of blood-brain barrier permeability. Examples of agonist compounds which initiate an increased permeability of the blood-brain barrier to an agent of interest include bradykinin, bradykinin analogues, other peptides and other compounds that mimic the increase in permeability of the blood-brain barrier in the manner elicited by bradykinin. Since these substances apparently interact with tissue-associated receptors to produce an increase in the permeability of the blood-brain barrier to an agent of interest, these substances can collectively be called "receptor mediated permeabilizers".

Bradykinin is a naturally occurring peptide comprised of nine amino acids having the following sequence: $NH_2$-Arginine-Proline-Proline-Glycine-Phenylalanine-Serine-Proline-Phenylalanine-Arginine-COOH (Lehninger, A.L., Biochemistry, p. 97 (1975)). A bradykinin analogue can be a structural derivative of bradykinin. Such analogues can be compounds which are derivatives of the number and/or sequence of amino acids in the bradykinin structure mentioned above which have a similar or enhanced effect on permeability of the blood-brain barrier. Bradykinin analogues also include modifications to the bradykinin molecule effected by changing or chemically modifying the normal amino acids, modifying peptide bonds, adding C-terminal and/or N-terminal extensions, etc.

A method for preparing bradykinin analogues is Merrifield's procedure of solid-phase peptide synthesis (Merrifield, R.B., J. Am. Chem. Soc., 86: 304 (1964); Draprau, G. and Regoli, D., Methods in Enzymology, 163: 263-272 (1988)). The first step in a solid-phase synthesis of bradykinin analogues is the formation of a covalent bond between the C-terminal protected amino acid of the chosen peptide sequences and the solid support or resin. The peptide chain is built up residue by residue by repetitive cycles of deprotection, during which the N-terminal Boc-protecting (N-tert-butoxycarbonyl) group is removed by trifluoroacetic acid (TFA). This is followed by neutralization with diisopropylethylamine (DIEA) of the amino group left as a salt and coupling of the next amino acid in the sequence. The cycle is repeated until the sequence is completed. After its complete assembly, the peptide is cleaved from the resin and purified.

Compounds that mimic the bradykinin-like increase in permeability of the blood-brain barrier can also be synthesized as polypeptides, purified from mixtures of naturally occurring polypeptides, be chemically modified forms of such polypeptides or be chemically synthesized structures that act as bradykinin agonists of blood-brain barrier permeability. The essential characteristic of these compounds is in their ability to elicit an increase in blood-brain barrier permeability in a manner similar to that of bradykinin.

An effective amount of a bradykinin agonist of blood-brain barrier permeability is that amount which will significantly increase the blood-brain barrier permeability for the agent of interest. In other words, it will increase the permeability of the blood-brain barrier to allow sufficient quantities of an agent to pass from the blood to the interstitial fluid of the brain to exert a therapeutic or prophylactic effect or allow diagnostic procedures. The effective amount will be determined on an individual basis and will be based, at least in part, on consideration of the patient's size, the specific disease, the severity of symptoms to be treated, the result sought and the specific bradykinin agonist of blood-brain barrier permeability used. Thus, the effective amount can be determined by one of ordinary skill in the art employing such factors and using no more than routine experimentation.

The increase in permeability of the blood-brain barrier in response to a bradykinin agonist relates not only to the quantity of agents passing from the blood to the interstitial fluid of the brain, but also, to the type of agent to be passed from the blood to the interstitial fluid of the brain. The effect of bradykinin agonists of blood-brain barrier permeability is to preferentially increase the passage of small molecular weight substances. For example, the data listed in Example VII demonstrate that the permeability of the blood-brain barrier of mice to a 40,000 molecular weight molecule was not substantially affected while the permeability of molecules with molecular weights of 1,000 or less was statistically significantly increased. Thus, while the permeability of small molecular weight substances is increased, the exclusion of the blood-brain barrier to substances of significantly higher molecular weight is substantially maintained. (See Example VII for further detail.)

Those skilled in the art will understand that mixtures of bradykinin analogues can also be administered as agonists of blood-brain barrier permeability. For example, bradykinin plus a bradykinin analogue, such as A-7, could be administered together. In like manner, A-7 could be administered with one or more other bradykinin analogues or with bombesin or VIP or with other agonists of blood-brain barrier permeability.

It will also be understood by those skilled in the art that the agonists of blood-brain barrier permeability should be non-toxic to the patient. Agonists are non-toxic if a therapeutically effective amount does not result in death to the patient.

In a specific application of the invention, a patient afflicted with toxoplasmosis encephalitis can be treated by administering an effective amount of an antiparasitic agent, such as clindamycin or gentamycin, and co-administering an angonist of blood-brain barrier permeability. The co-administration of the agonist of blood-brain barrier permeability allows passage of the antiparasitic agent through the blood-brain barrier to treat the parasitic infection.

The invention is further illustrated by the following specific examples.

Example I. Effect of Intravenous Co-Administration Bradykinin on Brain Tissue Uptake of Sucrose and Bovine Serum Albumin (BSA).

Female Balb/C mice weighing approximately 20g were employed. All solutions were prepared in sterile PBS. Intravenous injections (100 μl) were performed in the tail vein. $^3$H-sucrose ($10^6$ cpm) was injected as a bolus without or with bradykinin as stated. Mice were killed 10 minutes after injection. Blood was collected in heparinized tubes and a 100 μl aliquot was counted in a liquid scintillation counter after addition of 1 ml of 1% sodium dodecyl sulfate (SDS) to ensure solubilization of proteins, 300 μl 37% hydrogen peroxide as a bleaching agent, and 15 ml Aquasol-2 (Dupont). The brain was removed, homogenized in 5 ml $H_2O$, and 1 ml of the homogenate was aliquoted for liquid scintillation counting (after addition of 1 ml of 1% SDS and 15 ml Aquasol-2). Relative brain uptakes were calculated as the ratios of radioactivity recovered in brain over that recovered in blood, and expressed as [radioactivity in 1 μl blood] (μl/g)/(radioactivity in 1g brain]. In some experiments, $^{14}C$-BSA (5 x $10^5$ cpm) was co-injected together with $^3H$-sucrose ($10^6$ cpm), without or with bradykinin, and the relative brain uptakes of $^{14}C$-BSA and $^3H$-sucrose were calculated after double isotope scintillation counting.

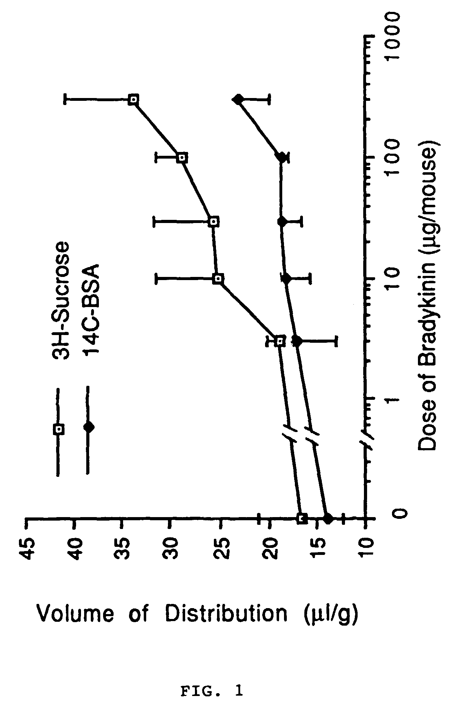

The results are displayed in Figure 1. As this Figure shows, the relative brain uptake of $^3H$-sucrose 10 minutes after injection was significantly increased when bradykinin was co-administered with $^3H$-sucrose at doses of 10, 30, 100 and 300 μg and reached a value of 33 μl/g. The threshold dose of bradykinin was 10 μg, while the two highest doses of 100 and 300 μg yielded almost identical increases in relative brain uptake of $^3H$-sucrose. In contrast, bradykinin at doses of up to 100 μg had marginal effect on the relative brain uptake of $^{14}C$-BSA, which remained between 14 and 18 μl/g. At a bradykinin dose of 300 μg, the relative brain uptake of $^{14}C$-BSA increased to 23 μl/g.

Example II. Pharmacological Characterization of the Effect of Bradykinin, Bradykinin Anologues and Other Peptides on the Mouse Blood-Brain Barrier.

The protocol for these experiments was the same as described in Example I, except as noted. Drugs were administered to the mice at the concentrations listed in Table 1. For bradykinin and bradykinin analogues, brain levels of $^3H$-sucrose were determined 10 minutes after treatment. For bombesin and vasoactive intestinal peptide, brain levels of $^{14}C$- sucrose were determined 10 minutes after treatment. The brain uptake data are expressed as mean ± s.d. and are derived from two independent experiments except for the desArg[9] - bradykinin, bombesin and vasoactive intestinal peptide results which were derived from one experiment for each peptide. Four mice were used for each different drug per experiment. The results are as follows:

Table I

| Treatment | Dose/ Mouse | N | Brain Uptake of sucrose (% Control) | % Increase over Control |
|---|---|---|---|---|
| None (Control) | | 8 | 100 ± 9 | 0 |
| Bradykinin | 300 μg | 8 | 177 ± 24 | 77 |
| [Phe[8]Ψ($CH_2$-NH)Arg[9])bradykinin | 125 μg | 8 | 174 ± 15 | 74 |
| N-acetyl[Phe[8]Ψ($CH_2$-NH)Arg[9]] bradykinin | 100 μg | 5 | 230 ± 26 | 130 |
| desArg[9] -bradykinin | 300 μg | 4 | 90 ± 15 | -10 |
| Bombesin | 10 μg | 4 | 207 ± 14 | 107 |
| Vasoactive Intestinal Peptide | 10 μg | 4 | 209 ± 7 | 109 |

Bradykinin significantly increased the brain uptake of $^3H$-sucrose. In addition, the bradykinin analogue and agonist [Phe[8]Ψ($CH_2$-NH)Arg[9]]bradykinin, which is believed to be specific for $B_2$ receptors, also increased brain uptake. This agonist differs from bradykinin in that the peptide bond between the 8th (Phe) and 9th (Arg) amino acids is replaced with the isosteric $CH_2$-NH bond in order to protect bradykinin from degradation. When this agonist is treated further with N-acetylation to give N-acetyl[Phe[8]Ψ($CH_2$-NH)Arg[9]] bradykinin, the effect on brain uptake is increased to a similar extent as that obtained with bradykinin or the original analogue. This form of the analogue is also devoid of histamine-releasing activity, unlike bradykinin itself. One product of bradykinin degradation is desArg[9]-bradykinin. This degradation byproduct results from the action of kininase 1 (carboxypeptidase N), a proteolytic enzyme, which removes the C-terminal arginine. This bradykinin analogue is believed to retain biological activity on $B_1$ receptors, but did not substantially affect the brain uptake of sucrose through the blood-brain barrier.

The peptides bombesin (p Glutamate-Glutamine-Arginine-Leucine-Glycine-Asparagine-Glutamine-Tryptophan-Alanine-Valine-Glycine-Histidine-Leucine-Methionine-$NH_2$) and vasoactive intestinal peptide (Histidine-Serine-Aspartate-Alanine-Valine-Phenylalanine-Threonine-Aspartate-Asparagine-Tyrosine-Threonine-Arginine-Leucine-Arginine-Lysine-Glutamine-Methionine-Alanine-Valine-Lysine-Lysine-Tryptophan-Leucine-Asparagine-Serine-Isoleu-

cine-Leucine-Asparagine-$NH_2$) significantly increased the brain uptake of $^{14}$C-sucrose. This indicates that these peptides are receptor mediated permeabilizers.

Example III. Synthesis of the Boc-4-MeTyr (ΨCH2NArg (Tos)-O-Resin

N-BOC-O-Methyl-L-Tyrosine N-Methoxy-N-methylamide

To 350 ml of anhydrous THF on ice was added 3.635g (37.2 mmols) of N,O-dimethylhydroxylamine hydrochloride. The mixture was stirred for 10 minutes to allow most of the N,O-dimethylhydroxylamine hydrochloride to dissolve. Then, the following ingredients were successively added to the flask: 10g (33.8 mmols) of N-Boc-O-methyl-L-tyrosine, 6.977g (33.8 mmols) of dicyclohexylcarbodiimide, 1.96g (16.04 mmols) of 4-dimethylaminopyridine, and 6.209ml (35.64 mmols) of N,N-diisopropylethylamine. When all of the reagents had been added, the reaction flask was placed in a cold room (4°C) and stirred for 12 hours. The contents of the flask were gravity filtered using Whatman Qualitative #1 filter paper. The filtrate was concentrated by means of a rotary evaporator to viscous oil which was then redissolved in 200 mls of methylene chloride. This crude reaction mix was allowed to sit at 4°C for one hour and then filtered as before in order to remove any residual dicyclohexylurea. The filtrate was again concentrated by means of a rotary evaporator and redissolved in 50 ml of methylene chloride in preparation for column chromatography. Column chromatography was performed using silica (230-400 mesh, 60A) as the adsorbent and 50/50 ethyl acetate/hexane as the eluant. The column used for this scale reaction was 70 cm in length and 10 cm in diameter. The product eluted after approximately 400 ml of eluant had been run through the column. The fractions were all checked by TLC using Silica Gel 60 F-254 glass backed plates. The desired product (Rf value of 0.46) was pooled and concentrated in vacuo. Concentration afforded clear, colorless oil which was placed under high vacuum for several hours. At the end of this time the product remained as a semi-solid material and with time became completely solid.

There remained 5.73 g (50.2%) of a white solid with a mp of 58-62°C; IR (cm$^{-1}$, KBr) 3320, 2980, 2840, 1710, 1655, 1520, 1205, 1180; MS mle 338.4 (M+); $^1$H(CDCl$_3$, 300 MHz) δ7.08 (d,2H, J-8.50 Hz), δ6.82 (d, 2H, J-8.50 Hz), δ5.15 (br d, 1H, J=8.90 Hz), δ4.89 (br m, 1H), δ3.78 (s, 3H), δ3.66 (s, 3H), δ3.17 (br s, 3H), δ2.99 (d of d, 1H, J=6.0 Hz), δ2.83 (d of d, 1H, J=6.0 Hz), δ1.39 (s, 9H); Anal. Calcd; C, 60.35; H, 7.69; N, 8.28. Found: C, 60.58; H, 8.02; N, 8.31.

N(t-Butoxycarbonyl)-O-methoxy-L-tyrosinal:

To 150 ml of anhydrous ethyl ether was added 1.04g (27.37) mmols of lithium aluminum hydride and the suspension was gently refluxed for 30 minutes. Upon cooling to 4°C, the reflux condenser was replaced by a pressure equalizing dropping funnel containing 7.4g (21.9 mmols) of N-(t-butoxycarbonyl)-O-methyl-L-tyrosine N-methoxy-N-methylamide dissolved in 100 ml of anhydrous ethyl ether. The contents of the funnel were added over one hour. The reaction mix was allowed to react for an additional two hours. At the end of this time a cold solution of KHSO$_4$ (5.433 g in 230 ml of H$_2$O) was added to the reaction vessel. The layers were separated and the aqueous layer was extracted three times with 150 mls of ether each time. The ether layers were combined and worked up as follows: washed three times with 200 mls of 3N HCl; washed three times with 200 mls of saturated sodium bicarbonate; washed three times with 200 mls of brine; and dried over magnesium sulfate, filtered , and concentrated in vacuo. There remained 3.78 g (61.6%) of a white solid with a mp of 69-72°C: Rf-0.65 in 50/50 ethyl acetate/hexane; IR (cm$^{-1}$, KBr) 3360, 2840, 1740, 1695, 1530, 1255, 1180: MS m/e 279.3 (M$^+$); $^1$H (CDCl$_3$), 300 MHz) δ9.63 (s, 1H), δ7.08 (d, 2H, J=8.5 Hz) δ6.84 (d, 2H, J=8.5), δ5.05 (br s, 1H), δ4.40 (m, 1H), δ3.79 (s, 3H), δ3.06 (d, 2H, J=6.50), δ1.44 (s, 9H); $^{13}$C NMR (CDCl$_3$, 75.47 MHz) δ200, 158.79, 130.28, 127.69, 114.27, 61.05, 55.29, 34.70, 28.26; Anal. Calcd: C, 64.51; H, 7.52; N, 5.01. Found: C,64.60, H, 7.86; N, 4.93.

N-BOC-4MeTyr(ΨCH2NH)Arg(Tos)-OH

To a flask containing 100 ml of Methanol:Acetic Acid (99:1) was added 4.92g (15 mmols) of N$^g$-Tosyl-arginine followed by 1.15g (18 mmol) of sodium cyanoborohydride. The reagents were stirred for 5 minutes followed by the addition of 4.46g of N-BOC-4-Me-Tyrosinal. After 30 minutes, an additional 1.15g (18 mmols) of sodium cyanoborohydride was added to the reaction vessel. Three additional portions of sodium cyanoborohydride were added at thirty minute intervals and the reaction was allowed to stir overnight. The reaction was worked-up by evaporating the solvent. The residue was dissolved in heptane and dried followed by dissolution in ether and drying. Water (200 ml) was added to the flask and the solid collected by filtration. TLC analysis revealed a homogenous product with an Rf of 0.35 in CHCl3:MeOH,4:1). NMR was consistent with the expected product.

N-BOC-4MeTyr(ΨCH2N[Z])Arg(Tos)-OH

To 2.14 g (3.61 mmole) of the pseudodipeptide above was added 1.65 g (19.6 mmole) of $NaHCO_3$ in 100 ml of 1:1 dioxane/water. Benzyl chloroformate (0.6 ml, 4 mmol) was added and the reaction was stirred overnight. The solvents were removed in vacuo leaving a gummy residue. The residue was suspended in 100 ml of water and this was acidified to pH2 with HCl and extracted three times with ethyl acetate. The combined ethyl acetate fractions were dried over magnesium sulfate, filtered and evaporated to provide 2.35g (90%) of the desired material as a crude amorphous white solid. Recrystallization from methylene chloride/hexane provided 2.18g (83%) of the product as a white solid.

Attachment of the Protected Pseudodipeptide to the Polystyrene Resin

The protected pseudodipeptide was attached to hydroxymethyl resin (Polystyrene-1% Divinylbenzene, 0.7 mequiv./g) using dicyclohexylcarbodiimide and 4-dimethylaminopyridine. To 1.87 g of hydroxymethyl resin (1.31 mmol) was added 2.28 g (3.28 mmol) of the protected pseudodipeptide, 410 mg (3.33 mmol) of 4-dimethylaminopyridine, and 25 ml of anhydrous dimethylformamide in a 50 ml polypropylene tube. To this was added 680 mg (3.3 mmol) of dicyclohexylcarbodiimide and the vessel was shaken overnight at room temperature. The resin was collected by filtration and washed successively three times each with methylene chloride and methanol and dried overnight in vacuo to provide 2.6g of resin. Substitution by weight gain was calculated to be 0.54 mmol/g.

Example IV. Synthesis and Purification of A-7

A-7 was prepared by solid-phase peptide synthesis by sequentially assembling the remaining amino acids (in a C- to N-fashion) onto the resin bound protected pseudodipeptide. The peptide was synthesized on a Beckman 990 peptide synthesizer using the following program for each coupling cycle, 1- Wash, $CH_2Cl_2$ (3 x 1 min); 2-Deprotect 40% TFA/$CH_2Cl_2$ (2 x 10 minutes); 3- Wash, $CH_2Cl_2$ (3 x 1 min); 4 - Wash. Isopropanol (2 x 1 min); 5 - Wash, $CH_2Cl_2$ (3 x 1 min); 6- Neutralize 5% DIEA/$CH_2Cl_2$ (3 x 2 min); 7 - Wash, $CH_2Cl_2$ (5 x 1 min); 8 - Couple (3 equivalents Boc-Amino acid, 3 equivalents of BOP) 1 x 60 min; 9 - Wash, $CH_2Cl_2$ (3 x 1 min); 10 - Wash, Isopropanol (2 x 1 min); 11 - Wash, $CH_2Cl_2$ (3 x 1 min; 12- Test for coupling by ninhydrin. If recoupling was necessary as judged by a positive ninhydrin test, a partial cycle starting at step 6 to the end was done. Following assembly of the complete protected peptide, the N-terminal BOC group was removed by using steps 1-5 in the cycle and the resin dried.

The crude peptide was isolated from the resin by treatment of the protected peptide-resin with anhydrous HF containing 10% anisole for 1 hour at 0°C. The HF was removed in vacuo and the crude resin/peptide was washed with ether three times. The peptide was extracted with 10% acetic acid and lyophilized to yield a crude peptide.

The peptide was partially purified by HPLC using a 0.1% TFA/acetonitrile gradient (10-40% over 30 minutes) on a $C_{18}$ reverse phase support. The peptide was further purified using isocratic 15% acetonitrile in 0.1% TFA as an eluant on a $C_{18}$ support. The fractions from the main peak were combined to provide purified A-7 which appeared homogeneous by TLC, Electrophoresis, and HPLC. FAB/MS analysis yielded the expected MW of 1098. Amino acid analysis often 6N HCl hydrolysis (24 h @ 110°C) gave the following composition: (Ser(1)0.89, Pro (2)2.00, Gly (1)0.97, Arg (1)1.03, Thi(1)0.73g 4Me-Tyr-(ΨCH₂NH)-Arg(1), detected by an alternate method, was partially destroyed in hydrolysis.

Example V. Time Course of Blood-Brain Barrier Opening in Mice and Rats.

Female Balb/C mice weighing approximately 20 g were use. All solutions were prepared in sterile phosphate-buffered saline. Intravenous injections of 100 μl of the bradykinin analogue and agonist [Hyp[3], Thi[5] ,4-Me-Tyr[8]Ψ(CH₂NH)Arg[9]]bradykinin (hereinafter designated as A-7) were given in the tail vein at time = 0. This agonist differs from bradykinin in that the 3rd (Pro) amino acid has been replaced by hydroxyproline, the 5th (Phe) amino acid has been replaced by thienylalanine, the 8th (Tyr) amino acid has been substituted with a methyl group at the 4 position and the peptide bond between the 8th (4-Me-Tyr) and 9th (Arg) amino acids is replaced with the isosteric CH₂-NH bond. At various specified times following the injection of A-7 an injection of $^{14}C$-sucrose ($3 \times 10^6$ dpm) was also made in the tail vein. Two, five or ten minutes later, the mice were killed. Blood was collected in heparinized tubes and centrifuged to separate plasma from the resulting pellet. A 100 μl aliquot of plasma was counted by liquid scintillation counting after the addition of 15 ml of Aquasol-2 (Dupont). The brain was removed and homogenized in 2.5 ml of water and 2.5 ml of 1% SDS. One ml of the homogenate was aliquoted and added to 15 ml of Aquasol-2 for counting. Brain uptake of sucrose was calculated and expressed as percent of injected dose X 100.

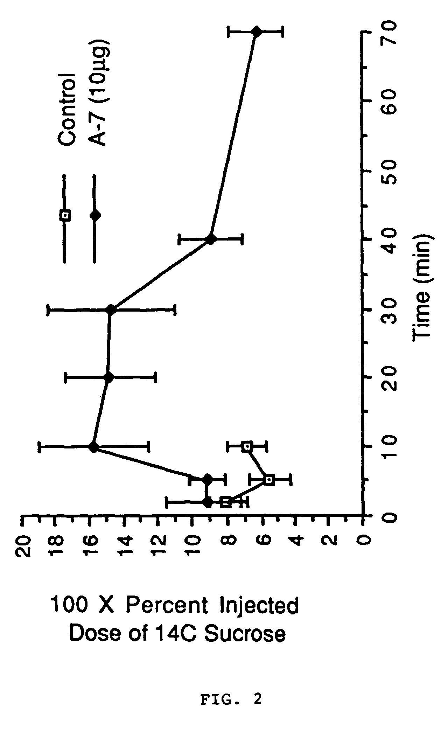

The results are displayed in Figure 2. As seen in this Figure, the uptake of $^{14}C$-sucrose at 10 and 20 minutes was significantly higher in the presence of A-7. The blood-brain barrier remained permeable to sucrose for at least 20 minutes following injection of A-7 but did not remain open after 40 minutes. Each data point represents the mean ± s.d.

from 8 mice.

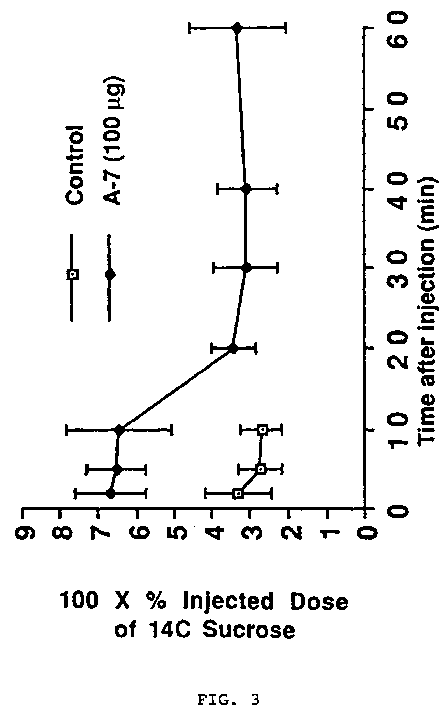

The time course of blood-brain barrier opening in rats is shown in Figure 3. The methodology is essentially the same as in the above description except that female Harlan Sprague-Dawley rats (150-200 g) were used and 6 X $10^6$ dpm of $^{14}$C-sucrose were used. The blood-brain barrier remains open to sucrose for 20 minutes in rats following A-7 injection. Each data point represents the mean $\pm$ s.d. from 6 rats.

In some experiments whole blood samples and intact brain tissue were combusted in a Packard Model 307 Oxidizer and the radioactivity collected and counted in a liquid scintillation counter. The results obtained by either homogenization or combustion of samples were equivalent.

Example VI. Dose Response Relationship of Bradykinin and Analogues. Sucrose Uptake Studies.

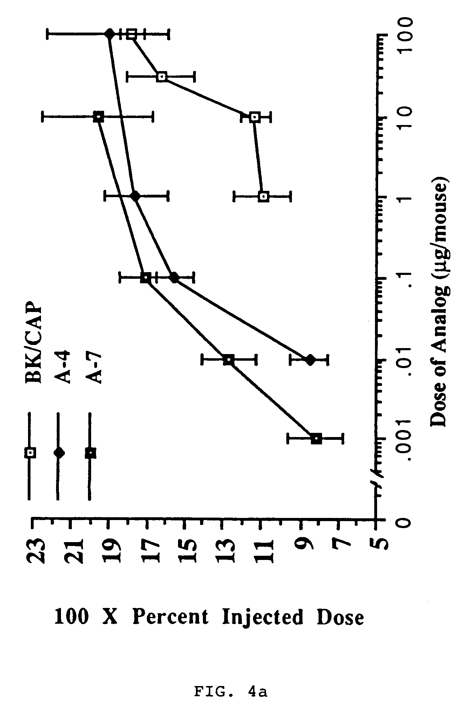

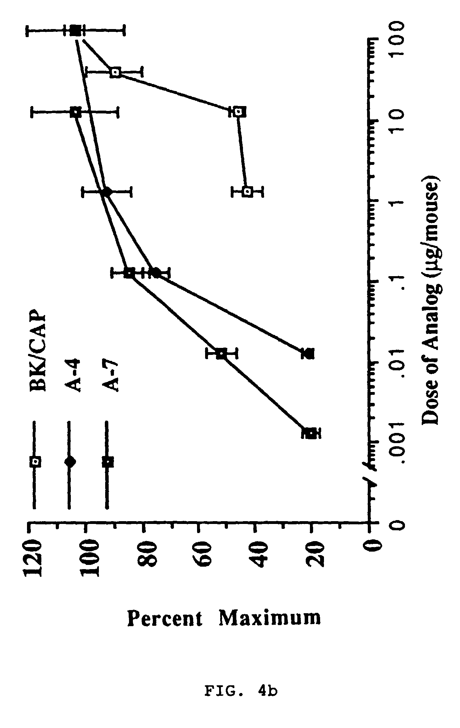

The methodology for these experiments is similar to the above two time course studies with the exception that all mice were killed 10 minutes after receiving a single tail vein injection of $^{14}$C-sucrose simultaneously with bradykinin (BK) or bradykinin analogue. Figure 4a shows the amount of $^{14}$C-sucrose uptake expressed as 100 X percent of injected dose over various concentrations of bradykinin and brandykinin analogues after 10 minutes. Figure 4b represents the percent maximum response (sucrose uptake) over the range of doses of bradykinin or bradykinin analogue shown. The bradykinin analogue and agonist ([Thi$^5$ ,Phe$^8\Psi$ (CH$_2$-NH) Arg$^9$]bradykinin) (A-4) and, particularly, [Hyp$^3$,The$^5$,4-Me-Tyr$^8\Psi$(CH$_2$NH)Arg$^9$]bradykinin (A-7) are more potent than the combination of bradykinin and captopril (BK+Cap). The number of mice per data point ranged from 12 to 16.

Example VII. Uptake of Substances of Different Molecular Weights into the Brain of Mice When Co-administered with A-7.

The methodology of these experiments is similar to that of the above two examples. Specific radioactively labeled molecules of different molecular weight and structure, either with saline or with 10 μg of A-7, were intravenously injected into mice via the tail vein. The animals were sacrificed 10 minutes after the coinjection and the radioactivity present in brain was measured as described previously for $^{14}$C sucrose. The results of these studies are shown in Table I.

TABLE I

| Uptake of Radiolabelled Substances into the Brain | | | |
|---|---|---|---|
| Molecule | Mol. Weight | 100 X % Injected Dose | |
| | | Control | + 10μg A-7 |
| $^{14}$C-Sucrose | 342 | 5.9±1.4 | 16.5±4.9 |
| $^3$H-Inulin | 5,000 | 0.075±0.018 | 0.110±0.016 |
| $^3$H-RNase | 12,600 | 0.123±0.039 | 0.117±0.030 |
| $^3$H-Myoglobin | 14,000 | 0.092±0.022 | 0.073±0.014 |
| $^3$H-Carbonic Anhydrase | 30,000 | 0.090±0.013 | 0.106±0.015 |
| $^3$H-Ovalbumin | 46,000 | 0.079±0.016 | 0.093±0.017 |
| $^3$H-Bovine Serum Albumin | 68,000 | 0.333±0.098 | 0.209±0.056 |
| Data are presented as mean $\pm$ s.d. for 7 to 15 mice in each group. | | | |

It appears that substances with greater molecular weights do not readily cross the blood-brain barrier when coinjected with A-7. This suggests that the blood-brain barrier permeabilizer characteristics of A-7 are restricted to lower molecular weight substances.

Example VIII. The Effect of Bradykinin and Analogue on the Brain Uptake and Antinociceptive Effect of Loperamide. Tail Flick Assay.

Study A: Bradykinin

Female Balb/C mice weighing approximately 20g were used. The tail flick assay was performed using a Tail Flick Apparatus model TF6 (Emdie Instruments, Maidens, VA). The intensity of the radiant heat was adjusted daily to yield a reaction time between 2 and 3.5 seconds in naive untreated mice. The cutoff time was set at 7.5 seconds. The tail withdrawal reaction time of mice was measured three times at 10 second intervals immediately prior to intravenous injections. These three values were averaged and taken as basal value ($V_O$). Another three measurements were taken 10 minutes after intravenous injections and averaged ($V_{10}$). In some experiments, the opiate receptor antagonist naloxone (NAL) was administered intraperitoneally 15 minutes (100 μl in sterile PBS, 10 mg/kg) prior to intravenous administration of bradykinin and loperamide (BK+LOP). The results were expressed as percent antinociceptive response according to the formula: $100 \times (V_{10} - V_O)/(7.5 - V_O)$ .

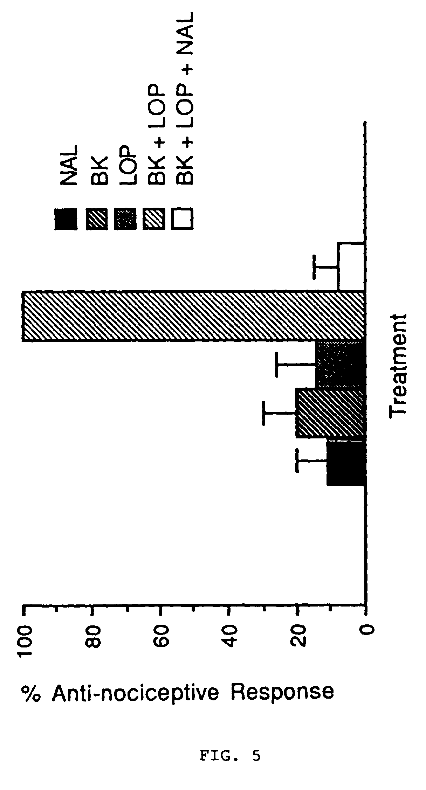

Figure 5 illustrates the effectiveness of bradykinin on the brain uptake of the opiate receptor agonist loperamide (LOP) by demonstration of antinociceptive effects. While loperamide injected intravenously at a dose of 25 μg per mouse had no activity, a complete antinociceptive response was observed when bradykinin (30 μg) was co-injected with the opiate, with the latency of tail withdrawal reaching the cut-off limit of 7.5 seconds in all mice. Pretreatment of mice with naloxone (10 mg/kg) completely antagonized this antinociceptive activity.

Study B: [Hyp3,Thi5,4-Me-Tyr8Ψ (CH2NH)Arg9]bradykinin

Female Balb/C mice weighing approximately 20 g were used. The tail flick assay was performed using a Tail Flick Apparatus model TF6 (Emdie Instruments, Maidens, VA). The intensity of the heat source was adjusted daily to yield a reaction time between 2.0 and 3.2 seconds in naive untreated mice. The maximum time of exposure to the heat source allowed was 7.5 seconds. The tail withdrawal reaction time of mice was measured 4 times at 10 second intervals immediately prior to intravenous injections via the tail vein. The last three values were averaged and taken as the baseline value ($V_0$). Another set of measurements was taken at the following intervals after tail vein injection: immediately, 5 min, 10 min, 15 min, 30 min, and 60 min. The last three values for each of these time points (V) was averaged. In some experiments the opiate receptor antagonist naloxone (10 mg/kg; 100 μl in saline) was administered intraperitoneally 15 minutes prior to administration of A-7 (0.1 μg) and loperamide (25 μg). The results were expressed as percent antinociceptive response according to the formula: $100 \times (V-V_0)/(7.5-V_0)$ .

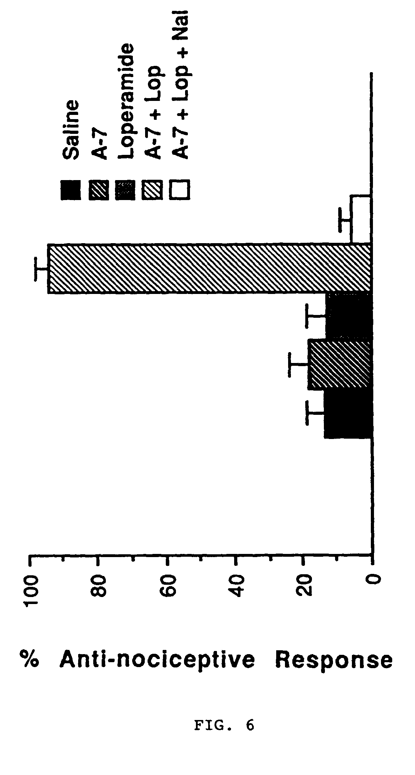

Figure 6 illustrates the ability of A-7 to enhance the permeability of the blood-brain barrier to loperamide as evidenced by the increase in % antinociceptive response. Each point represents pooled data from 2 experiments with 4 mice (total of 8 mice) 30 minutes after injection of loperamide, A-7, and A-7 and loperamide with or without naloxone pretreatment. A complete antinociceptive response was obtained when A-7 was coinjected with loperamide. The effect was completely antagonized by pretreatment with naloxone.

Example IX. The Effect of the Dopaminergic Agent Diacetyldopamine When Co-Administered with Bradykinin on the Locomotor Activity of Rats.

Five Sprague-Dawley rats (125-150g) were habituated to activity cages two days prior to the experiment. The activity cages were standard size rat cages with two photocell beams running across the floor of each cage. Any movement of the rat through either of the beams was recorded by a computer. Locomotor activity was measured as the number of beam breaks in a sequence of 10-minute intervals over two hours.

On test days, all rats were pretreated with the monoamine oxidase inhibitor nialamide (100 mg/kg i.p.). This was done to prevent the enzymatic degradation of diacetyldopamine (AcDA) by monoamine oxidases. They were then placed in the activity cages to habituate. Two hours later, rats were briefly removed from the cages and injected intravenously via tail vein injection with either AcDA (2mg) or AcDA (2mg) + bradykinin (BK) (1mg). The rats were immediately returned to the activity cages and locomotor activity was recorded over 10 minute intervals for the next two hours.

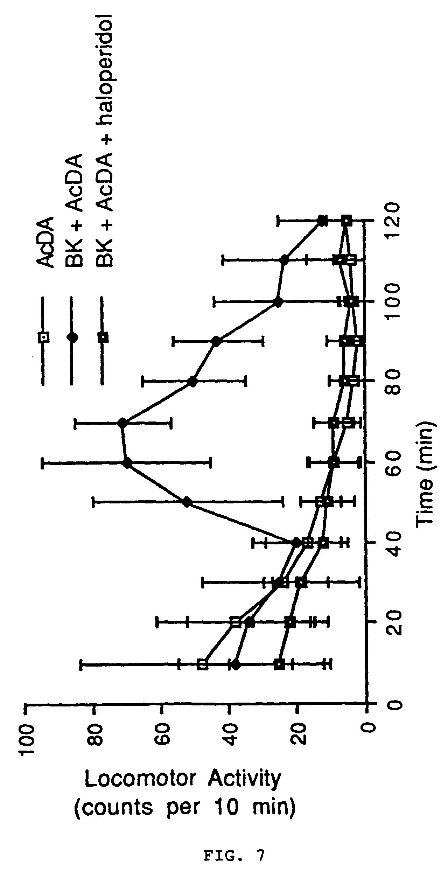

The results are displayed in Figure 7. As this Figure shows, those rats receiving AcDA alone showed no increase in locomotor activity during the two hour test session. However, when AcDA + bradykinin were coinjected, there was a significant increase in the locomotor behavior of the rats. These data suggest that bradykinin had enabled AcDA to enter the brain resulting in stimulation of central nervous system dopamine receptors.

In order to verify that the motor increase seen was due to direct stimulation of dopamine receptors, one group of rats received the dopamine antagonist haloperidol in addition to AcDA + bradykinin. The haloperidol (0.2 mg/kg i.p.) was administered 15 minutes prior to the AcDA/bradykinin injection. As can be seen in the figure, the increased motor activity was completely abolished with the haloperidol pretreatment.

Example X. The Effect of a Dopaminergic Antagonist When Co-administered with A-7 on the Locomotor Activity of Rats.

Domperidone is a dopamine receptor antagonist used clinically as an anti-emetic by virtue of its action at the area postrema outside the blood-brain barrier. Reports in the literature demonstrated that domperidone does not cross the blood-brain barrier but when given as an injection into the cerebral ventricles, it effectively blocks binding of dopaminergic compounds, such as apomorphine, to dopamine receptors. A pertinent test is whether domperidone can antagonize a dopamine receptor agonist-induced increase in motor activity when coadministered with A-7 but is ineffective when administered without A-7.

Sprague-Dawley rats (125-150g) were habituated for two days to activity cages. The activity cages were standard size rat cages with two photocell beams running across the floor of each cage. Any movement of the rat through either of the beams was recorded by a computer. Locomotor activity was measured as the number of beam breaks in a sequence of 10-minute intervals over a two hour period.

The rats were given a coinjection of 10μg A-7 and 300μg domperidone, or the domperidone alone, one hour before a subcutaneous injection of apomorphine (0.75mg/kg), which is a dopamine agonist. The motor activity of the rats was measured in activity cages over 10-minute intervals for up to 2 hours post-apomorphine injection.

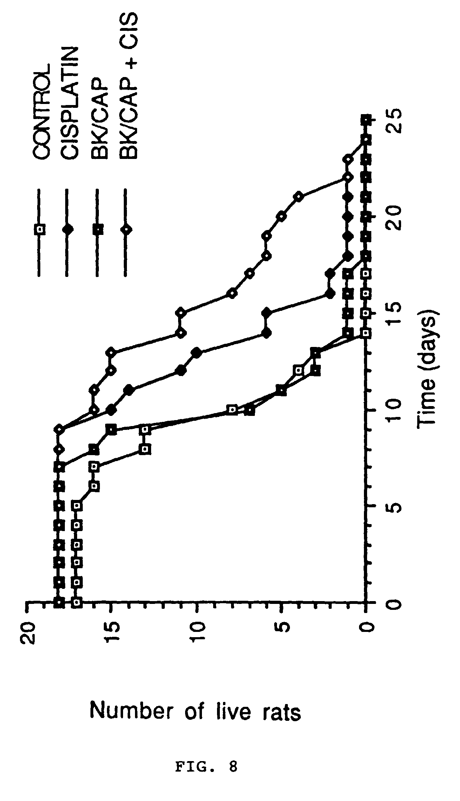

The results of this experiment with 3 rats in each treatment group are shown in Figure 8. The combination of A-7 and domperidone antagonized the increase in motor activity associated with apomorphine. Domperidone alone had little, if any, effect on motor activity induced by apomorphine which readily crosses the blood-brain barrier.

Example XI. The Effect of the Anti-Tumor Agent Cisplatin When Co-Administered with Bradykinin and Captopril or a Bradykinin Analogue on the Survival Time of Rats with Brain Tumor Implants.

Male Fisher 344 rats (200-250g) were anesthetized with ketamine HCl (100 mg/kg) and acepromazine (10 mg/kg). The animals were placed in a stereotaxic frame. The heads of the animals were shaven and a midline incision was made to expose the skull. A small hole was drilled over the right sensorimotor cortex. Lastly, a 100 μl cell suspension (9L glioma cells, $2.5 \times 10^6$ cells/ml) was injected over 5 minutes into the right caudate putamen of each animal and the scalp sutured. On the days that followed, the animals received treatments as described below. From day 4 until the end of the study or time of death, animals were observed daily for signs of failing health. When signs of very poor health were observed (eye hemorrhage, loss of righting reflex), animals were killed and perfused with paraformaldehyde for subsequent histology to verify the existence of the tumor.

Study A

Rats were treated on days 4 and 6 after implantation of the tumor with 600 μg cisplatin i.v. via the tail vein (18 rats, Cis), or 1 mg of captopril i.p. followed 15 minutes later by 1 mg bradykinin i.v. (18 rats, Cap-Bk), or 1 mg captopril i.p. followed 15 minutes later by 1 mg bradykinin i.v. followed immediately thereafter by 600 μg cisplatin i.v., (18 rats, Cis/cap-bk). Another group of rats received no treatment (17 rats, control).

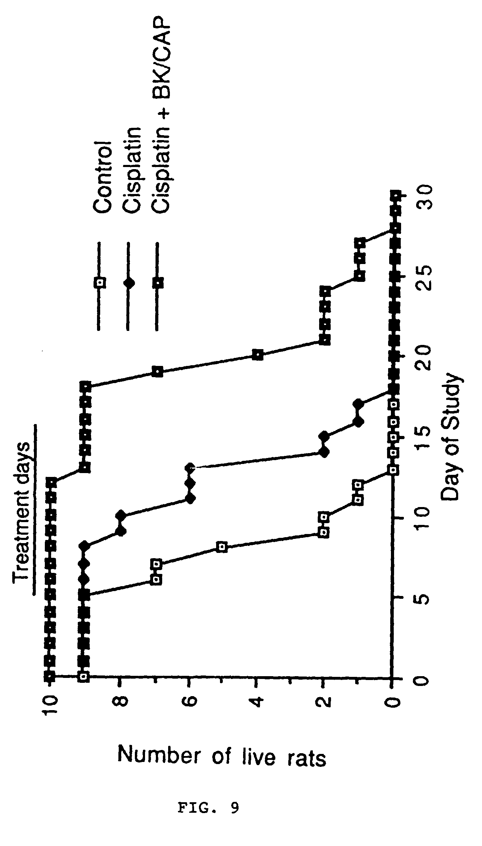

The results are shown in Figure 9, where survival time versus number of live rats is plotted. Treatment with captopril plus bradykinin had no effect on survival time. Treatment with the antineoplastic agent, cisplatin, did however significantly increase survival time and pretreatment with captopril and bradykinin before administering cisplatin significantly augmented this effect. The curves were fitted assuming they follow a Weibull distribution. The mean survival times and corresponding standard deviations are given in the following table in days:

TABLE II

| Group | Mean | S.D. |
|---|---|---|
| Control | 10.0 | 2.37 |
| Cisplatin | 13.1 | 2.90 |
| Captopril-Bradykinin | 10.0 | 1.84 |
| Cisplatin/captopril-Bradykinin | 16.5 | 5.77 |

When the anti-tumor agent, cisplatin, was administered alone, the survival time increased from 10.0 to 13.1 days. However, when cisplatin was co-administered with bradykinin and captopril, the survival tine increased even further to 16.5 days. These data suggest that the pretreatment of bradykinin (with captopril to prevent its degradation) increases the blood-brain barrier's permeability to cisplatin and thereby increases the anti-tumor agent's effectiveness in the brain. The Cis/Cap-Bk group was statistically significantly different from the Cis group with a p value less than 0.025.

Study B

Rats were treated daily from day 4 to day 14 after implantation of the tumor with 200 μg cisplatin i.v. (9 rats, Cis) or 1 mg captopril i.p. followed 15 minutes later by 1 mg bradykinin i.v. plus 200 μg cisplatin i.v. (10 rats, Cis/Cap-Bk). Another group received captopril and bradykinin, without cisplatin (9 rats, control).

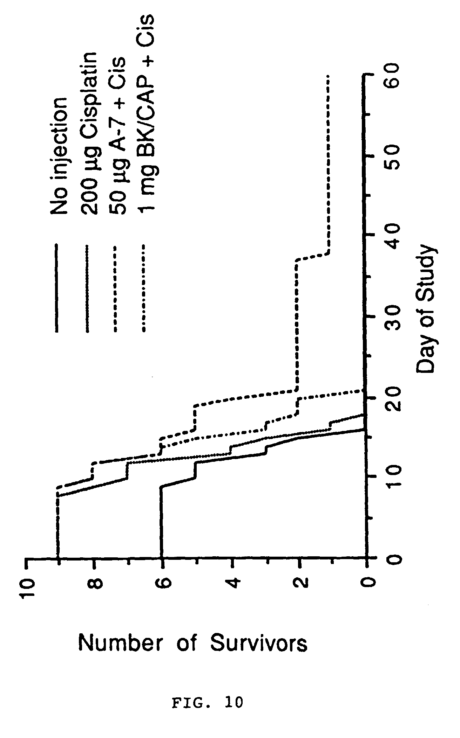

The results are shown in Figure 10 and confirm the earlier results in Study A which demonstrated that pretreatment with bradykinin and captopril significantly increase the effect of cisplatin on the survival time of rats with brain tumor implants. The mean survivals and corresponding standard deviations are given below in days:

TABLE III

| Group | Mean | S.D. |
|---|---|---|
| Control | 8.1 | 2.05 |
| Cisplatin | 11.9 | 2.62 |
| Cisplatin/Captopril-Bradykinin | 19.7 | 2.45 |

The survival times of rats receiving cisplatin with bradykinin and captopril were significantly higher than rats receiving only cisplatin. The Cis/Cap-Bk group was statistically different from the Cis group with a p value less than 0.005. The increased permeability of the blood-brain barrier caused by the administration of bradykinin (with captopril to prevent its degradation), apparently increased the brain uptake of cisplatin and thereby increased its therapeutic effectiveness.

Study C: [Hyp3,Thi5,4-Me-Tyr8Ψ(CH2NH)Arg9]bradykinin

Male Fisher 344 rats (200-250 g) were anesthetized with ketamine HCL (100 mg/kg) and acepromazine (10 mg/kg). The animals were placed in a stereotaxic device. The heads of the animals were shaved and a midline incision was made to expose the skull. A small hole was drilled over the right sensorimotor cortex. A 100 μl cells suspension (250,000 9L Glioma cells) was injected over 5 minutes into the right caudate putamen of each animal and the scalp sutured. Animals were observed daily for signs of failing health. When signs of very poor health were observed (eye hemorrhage or loss of righting reflex) animals were killed and the brains examined for presence of tumor.

On days 5 through 14, the animals received the following tail vein intravenous treatments: no treatment; cisplatin 200 μg/rat; A-7 (50 μg) and 5 minutes later cisplatin; or captopril pretreatment followed by 1 mg bradykinin 15 minutes later and cisplatin 5 minutes after bradykinin. The results are shown in Table IV as median with range.

TABLE IV

| Treatment Group | Median Survival (days) | No. Animals |
|---|---|---|
| Control | 14, Range 10-16 | 6 |
| Cisplatin | 13, Range 9-18 | 9 |
| BK & Captopril + Cisplatin | 16, Range 10-21 | 9 |
| A-7 + Cisplatin | 20.5, Range 10-62 | 9 |

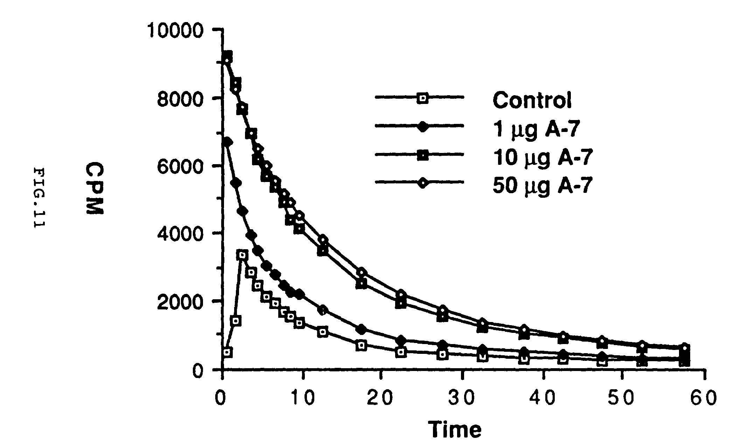

Figure 11 illustrates the survival times of all animals in the study. It should be noted that 2 animals in the A-7 + cisplatin treatment group had extended survival times, with one animal dying on day 38 and the other sacrificed at day 62. Both animals had evidence of tumor growth.

Example XII Effect of Bradykinin on the Brain Delivery of Various Molecules

The protocol for these experiments was the same as described in Example I, except as noted. Radiolabeled compounds (i.e., molecules) were administered intravenously to the mice with or without 300 μg of bradykinin. Brain levels were determined 10 minutes following treatment. The data are derived from 2-3 independent experiments for each compound. Four mice were used for each different molecule per experiment. The results were as follows:

Table V

| Molecule | Molecular Wt. Daltons | Increase in Brain Uptake (µl/g) | Percent Increase of Control |
|---|---|---|---|
| $^3$H-Sucrose | 342 | +7.5 ± 2.5* | 77 ± 24%* |
| $^3$H-AZT | 267 | +6.5 ± 2.6* | 34 ± 10%* |
| $^{14}$C-N-acetyl-Amphotericin B | 1,000 | +2.9 ± 0.9* | 24 ± 7%* |
| $^{14}$C-CD-4 | 40,000 | +1.9 ± 1.3 | 19 ± 13% |

\* Statistically significantly higher than brain uptake without bradykinin.

Bradykinin significantly increased the brain uptake of sucrose, AZT and N-acetyl-Amphotericin B. At a molecular weight of 40,000, however, the uptake of CD-4 was not significantly affected. The data indicate that bradykinin preferentially increases the blood-brain barrier permeability to small molecular weight substances.

Example XIII. 99mTc-DISIDA (N-[2,6-Diisopropylacetanilide]iminodiacetic acid) Uptake into the Head Region (Brain) in Rats.

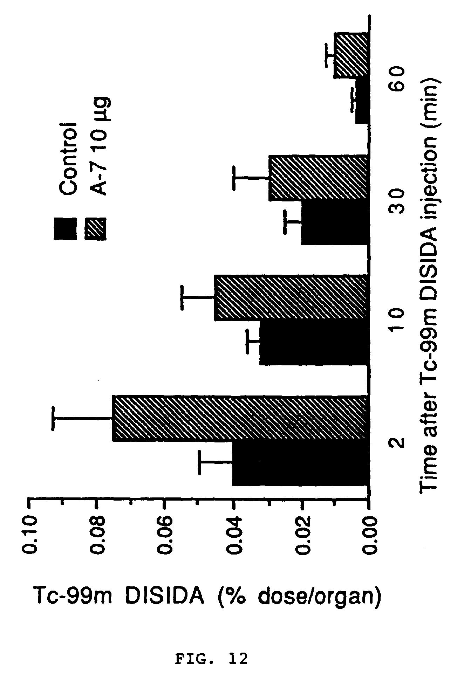

Female Sprague-Dawley rats (250-300g) were anesthetized with pentobarbital (60 mg/kg) and ketamine (100 mg/kg). The femoral veins were surgically exposed and the right vein was injected either with saline or a range of A-7 concentrations. After three minutes, a bolus of $^{99m}$Tc-DISIDA was injected into the left femoral vein. The rats were immediately placed on a gamma camera and the radioactivity counted intitially at 1 minute intervals and then at 5 minute intervals for 1 hour. The head region where the brain is the primary organ was identified and the amount of radioactivity present in this region is shown in Figure 12 for each of the concentrations of A-7 tested. The data for each concentration are radioactivity measurements from a single rat. At very early times the A-7 enhanced the uptake of [99m]TC-DISIDA into this region relative to the control animal. This experiment is representative of two similar studies.

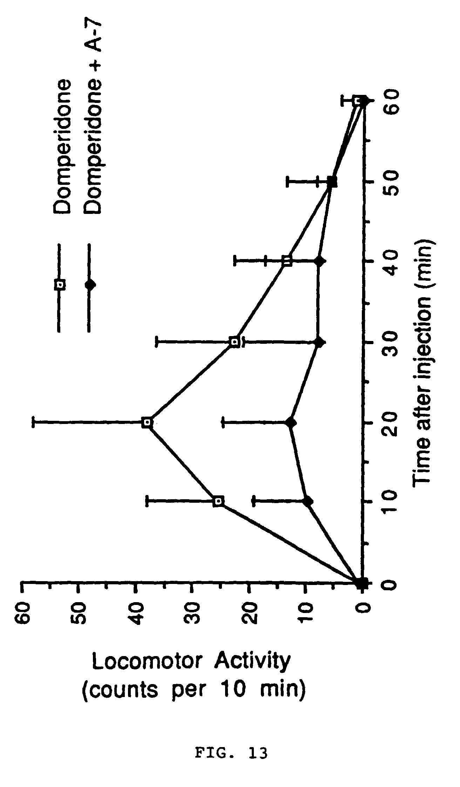

In another set of experiments, a single intravenous injection of A-7 was given into a femoral vein of an anesthetized rat. Two minutes later, an injection of $^{99m}$Tc-DISIDA was given into the contralateral femoral vein. In control animals, no A-7 was injected (sham injection). At time intervals of 2, 10, 30, or 60 minutes after the $^{99m}$Tc-DISIDA injection, the rats were sacrificed, their brains removed and counted in a gamma counter. The brain uptake of $^{99m}$Tc-DISIDA was calculated and expressed as percent of injected dose per organ. The biodistribution of $^{99m}$Tc-DISIDA in the whole brain of untreated and A-7 treated rats at selected times post-injection is shown in Table VI and Figure 13.

TABLE VI

| Biodistribution of $^{99m}$Tc-DISIDA in Brain | | |
|---|---|---|
| Time (after $^{99m}$Tc-DISIDA Injection) | % Injected Dost/Brain | |
| | Control | + 10µg A-7 |
| 2 min. | 0.040±0.013 | 0.075±0.019 |
| 5 min. | 0.032±0.003 | 0.046±0.006 |
| 10 min. | 0.022±0.005 | 0.028±0.003 |
| 60 min. | 0.004±0.001 | 0.010±0.003 |
| The data are expressed as mean ± s.d. for three animals per group. | | |

These results demonstrate that larger amounts of $^{99m}$Tc-DISIDA are found in the brain of A-7 treated rats when compared to control rats at early times postinjected of the labeled agent.

Example XIV. The Effect of Angiotensin II When Co-administered with A-7 on the Drinking Behavior of Rats.

Angiotensin II in supra-physiological concentrations has been shown to induce drinking behavior in water satiated rats. This behavior has been suggested to occur as a result of stimulation of angiotensin II receptors within areas of the brain not associated with the cerebroventricular organs. Studies were performed to evaluate the effect of co-administration of A-7 with an angiotensin II analogue that is capable of causing drinking behavior when administered at a high dose.

Rats were given a coinjection of 10μg A-7 and either 0.1, 0.3, 3, 10 or 30μg of β-angiotensin II, or the β-angiotensin II, alone, via tail vein injection. The volume of water consumed by each rat over a 1 hour interval was measured.

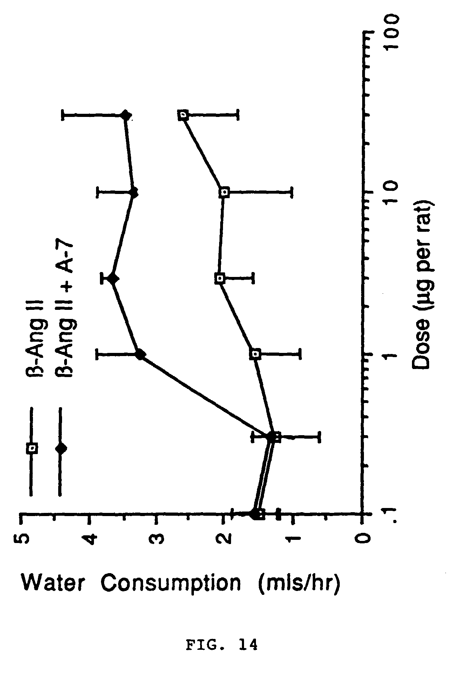

The results of this study with 6 rats in each dosage group are shown in Figure 14. Co-administration of A-7 and the angiotensin II analogue caused the dose response curve to shift to the left, or toward lower doses of analogue, when compared to administration of the analogue alone.

In another study, rats were given either saline, 1 μg of β-angiotensin II, 1 μg of β-angiotensin II and 10 μg of A-7, 1μg of β-angiotensin II and 8 μg of saralasin or 1 μg pf β-angiotensin II plus 10 μg of A-7 plus 8 μg of saralasin via tail vein injection. The saralasin was given because it is a known angiotensin II receptor antagonist. Again, the volume of water consumed by each rat over a 1 hour interval was measured.

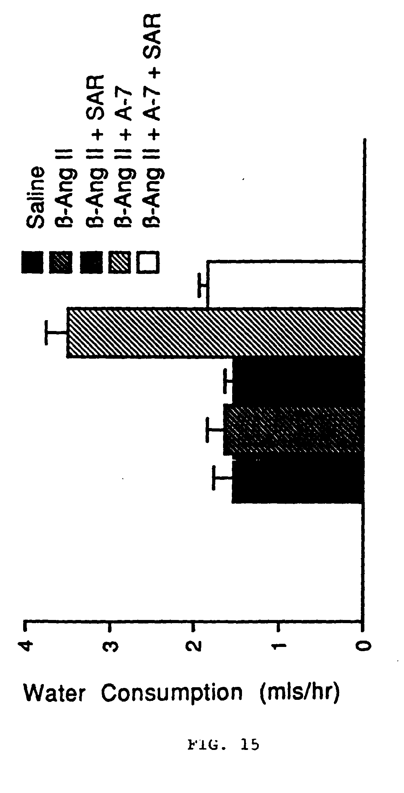

The results of this study with 3 rats in each group are shown in Figure 15. The co-administration of A-7 and β-angiotensin II causes a significant increase in water consumption compared to the angiotensin II analogue alone or together with saralasin. When saralasin is co-administered with A-7 and the analogue, the water consumption remains within a normal range which indicates an inhibition of angiotensin II analogue-induced drinking behavior by the addition of the angiotensin II receptor antagonist.

**Claims**

1. Use of a bradykinin agonist of blood-brain barrier permeability for the manufacture of a medicament for intravenous co-administration to a patient (e.g. a human being) for the purpose of increasing the permeability of the blood-brain barrier to a therapeutic or diagnostic imaging agent present in the blood stream of the patient.

2. Use according to claim 1, wherein said medicament comprises said agent, for simultaneous intravenous co-administration with said bradykinin agonist.

3. Use according to claim 1 or claim 2, wherein the bradykinin agonist of blood-brain barrier permeability comprises bradykinin, or a bradykinin analogue.

4. Use according to claim 3, wherein said bradykinin analogue has significantly increased resistance to proteolytic degradation in the blood stream of the human being relative to bradykinin.

5. Use according to claim 1 or claim 2, wherein the bradykinin agonist of blood-brain barrier permeability comprises compounds that mimic the increase in permeability of the blood-brain barrier in the manner of bradykinin.

6. Use according to claim 1 or claim 2, wherein the bradykinin agonist of blood-brain barrier permeability is selective for bradykinin 2 ($B_2$) receptors.

7. Use according to any one of the preceding claims wherein the agent is a diagnostic imaging agent, which for example is radiolabelled or doped with a magnetic resonance imaging contrast substance.

8. Use of a blood-brain barrier permeabilizer comprising bombesin vasoactive intestinal peptide (VIP), which increases the permeability of the blood-brain barrier in the manner of bradykinin for the manufacture of a medicament for intravenous co-administration to a patient (e.g. a human being) for the purpose of increasing the permeability of the blood-brain barrier to a therapeutic or diagnostic imaging agent present in the blood stream of the patient.

9. Use according to claim 8, wherein said medicament comprises said agent, for simultaneous intravenous coadministration with said blood-brain permeabilizer.

10. Use according to any one of claims 1 to 6, 8 or 9, wherein the agent is a neuropharmaceutical agent.

11. Use according to claim 10, wherein said neuropharmaceutical agent is any one of cisplatin, amphatericin B, azidothymidine, clindamycin, and gentamicin.

12. Use according to any one of claims 1 to 6, 8 or 9, wherein the agent is a therapeutic agent.

13. Use according to any one of claims 1 to 6, 8 or 9, wherein the medicament is for use in treating toxoplasmosis encephalitis and said agent is an anti-parasitic agent e.g. gentamicin or clindamycin.

14. A medicament consisting essentially of an anti-parasitic agent (e.g. gentamicin or clindamycin) and a bradykinin analogue which is a bradykinin agonist of blood-brain barrier permeability for intravenous co-administration in treating toxoplasmosis encephalitis.

15. Use of

    a) a therapeutic or diagnostic imaging agent to be delivered from the blood to the brain;
    b) a bradykinin agonist of blood-brain barrier permeability; and
    c) a pharmaceutically acceptable carrier for the manufacture of a pharmaceutical composition for intravenous co-administration to a human for the purpose of increasing the permeability of the blood-brain barrier to said agent.

**Patentansprüche**

1. Verwendung eines Bradykinin-Agonisten der Blut-Hirn-Schranken-Durchlässigkeit zur Herstellung eines Medikaments zur intravenösen Mitverabreichung an einen Patienten (z.B. einen Menschen) zwecks Steigerung der Durchlässigkeit der Blut-Hirn-Schranke für ein therapeutisches oder diagnostisches bildgebendes Agens im Blutstrom des Patienten.

2. Verwendung gemäß Anspruch 1, worin besagtes Medikament besagtes Agens zur gleichzeitigen intravenösen Mitverabreichung mit besagtem Bradykinin-Agonisten umfasst.

3. Verwendung gemäß Anspruch 1 oder Anspruch 2, worin der Bradykinin-Agonist der Blut-Hirn-Schranken-Durchlässigkeit Bradykinin oder ein Bradykinin-Analogon umfasst.

4. Verwendung gemäß Anspruch 3, worin besagtes Bradykinin-Analogon eine signifikant gesteigerte Resistenz gegen einen proteolytischen Abbau im Blutstrom des Menschen bezogen auf Bradykinin besitzt.

5. Verwendung gemäß Anspruch 1 oder Anspruch 2, worin der Bradykinin-Agonist der Blut-Hirn-Schranken-Durchlässigkeit Verbindungen umfasst, welche die Steigerung der Durchlässigkeit der Blut-Hirn-Schranke in gleicher Weise wie Bradykinin nachahmen.

6. Verwendung gemäß Anspruch 1 oder Anspruch 2, worin der Bradykinin-Agonist der Blut-Hirn-Schranken-Durchlässigkeit selektiv für Bradykinin 2 ($B_2$) Rezeptoren ist.

7. Verwendung gemäß einem der vorausgehenden Ansprüche, worin das Agens ein diagnostisches bildgebendes Agens ist, das zum Beispiel radiomarkiert oder mit einer Kernspintomografie-Kontrastsubstanz dotiert ist.

8. Verwendung eines Blut-Hirn-SchrankenDurchlässigkeitsförderers, der Bombesin vasoaktives Intestinalpeptid (VIP) umfasst, das die Durchlässigkeit der Blut-Hirn-Schranke in gleicher Weise wie Bradykinin steigert, zur Herstellung eines Medikaments zur intravenösen Mitverabreichung an einen Patienten (z.B. einen Menschen) zwecks Steigerung der Durchlässigkeit der Blut-Hirn-Schranke für ein therapeutisches oder diagnostisches bildgebendes Agens, das im Blutstrom des Patienten vorhanden ist.

9. Verwendung gemäß Anspruch 8, worin besagtes Medikament besagtes Agens zur gleichzeitigen intravenösen Mitverabreichung mit besagtem Blut-Hirn-Durchlässigkeitsförderer umfasst.

10. Verwendung gemäß einem der Ansprüche 1 bis 6, 8 oder 9, worin das Agens ein neuropharmazeutisches Agens ist.

11. Verwendung gemäß Anspruch 10, worin besagtes neuropharmazeutisches Agens ein beliebiges von Cisplatin, Amphatericin B, Azidothymidin, Clindamycin und Gentamicin ist.

**12.** Verwendung gemäß einem der Ansprüche 1 bis 6, 8 oder 9, worin das Agens ein therapeutisches Agens ist.

**13.** Verwendung gemäß einem der Ansprüche 1 bis 6, 8 oder 9, worin das Medikament für die Verwendung zur Behandlung von Toxoplasmose-Enzephalitis ist, und besagtes Agens ein antiparasitisches Agens, z.B. Gentamicin oder Clindamycin, ist.

**14.** Ein Medikament, das im wesentlichen aus einem antiparasitischen Agens (z.B. Gentamicin oder Clindamycin) und einem Bradykinin-Analogon, das ein Bradykinin-Agonist der Blut-Hirn-Schranken-Durchlässigkeit ist, besteht, zur intravenösen Mitverabreichung bei der Behandlung von Toxoplasmose-Enzephalitis.

**15.** Verwendung

    a) eines therapeutischen oder diagnostischen bildgebenden Agens, das vom Blut zum Gehirn überbracht werden soll;
    b) eines Bradykinin-Agonisten der Blut-Hirn-Schranken-Durchlässigkeit; und
    c) eines pharmazeutisch geeigneten Trägers zur Herstellung einer pharmazeutischen Zusammensetzung zur intravenösen Mitverabreichung an einen Menschen zwecks Steigerung der Durchlässigkeit der Blut-Hirn-Schranke für besagtes Agens.

**Revendications**

**1.** Utilisation d'un agoniste du type de la bradykinine vis-à-vis de la perméabilité de la barrière hémato-encéphalique pour la fabrication d'un médicament destiné à être co-administré par voie intraveineuse à un patient (par exemple un être humain) dans le but d'accroître la perméabilité de la barrière hémato-encéphalique à un agent thérapeutique ou d'imagerie de diagnostic présent dans le flux sanguin du patient.

**2.** Utilisation selon la revendication 1, dans laquelle ledit médicament comprend ledit agent en vue d'une co-administration par voie intraveineuse conjointement audit agoniste de type bradykinine.

**3.** Utilisation selon la revendication 1 ou la revendication 2, dans laquelle ledit agoniste du type bradykinine vis-à-vis de la perméabilité de la barrière hémato-encéphalique comprend de la bradykinine ou un analogue de la bradykinine.

**4.** Utilisation selon la revendication 3, dans laquelle ledit analogue de la bradykinine présente une résistance nettement améliorée à la dégradation protéolytique dans le flux sanguin de l'être humain par rapport à la bradykinine.

**5.** Utilisation selon la revendication 1 ou la revendication 2, dans laquelle l'agoniste de type bradykinine vis-à-vis de la perméabilité de la barrière hémato-encéphalique comprend des composés qui permettent, à la manière de la bradykinine, une augmentation de la perméabilité de la barrière hémato-encéphalique.

**6.** Utilisation selon la revendication 1 ou la revendication 2, dans laquelle l'agoniste de type bradykinine vis-à-vis de la perméabilité de la barrière hémato-encéphalique est sélectif vis-à-vis des récepteurs de la bradykinine 2 ($B_2$).

**7.** Utilisation selon l'une quelconque des revendications précédentes, dans laquelle l'agent est un agent d'imagerie de diagnostic, qui par exemple est radiomarqué ou dopé avec une substance pour le contraste en imagerie par résonance magnétique.

**8.** Utilisation d'un agent de perméabilité de la barrière hémato-encéphalique comprenant de la bombésine, le peptide vasoactif intestinal (VIP), lequel augmente la perméabilité vis-à-vis de la barrière hémato-encéphalique à la façon de la bradykinine pour la préparation d'un médicament destiné à être co-administré par voie intraveineuse à un patient (par exemple un être humain) dans le but d'accroître la perméabilité de la barrière hémato-encéphalique d'un agent thérapeutique ou d'imagerie de diagnostic présent dans le flux sanguin d'un patient.

**9.** Utilisation selon la revendication 8, dans laquelle ledit médicament comprend ledit agent en vue d'une co-administration par voie intraveineuse conjointement audit agent de perméabilité hémato-encéphalique.

**10.** Utilisation selon l'une quelconque des revendications 1 à 6, 8 ou 9, dans laquelle ledit agent est un agent neuropharmaceutique.

**11.** Utilisation selon la revendication 10, dans laquelle ledit agent neuropharmaceutique est choisi parmi le cisplatine, l'amphatéricine B, l'azidothymidine, la clindamycine, et la gentamicine.

**12.** Utilisation selon l'une quelconque des revendications 1 à 6, 8 ou 9, dans laquelle ledit agent est un agent thérapeutique.

**13.** Utilisation selon l'une quelconque des revendications 1 à 6, 8 ou 9, dans laquelle ledit médicament est destiné au traitement de la toxoplasmose encéphalitique et ledit agent est un agent anti-parasitaire tel que la gentamicine ou la clindamycine.

**14.** Médicament consistant essentiellement en un agent anti-parasitaire (par exemple la gentamicine ou la clindamycine) et en un analogue de la bradykinine qui est un agoniste du type de la bradykinine vis-à-vis de la perméabilité de la barriére hémato-encéphalique en vue d'une co-administration par voie intraveineuse dans le traitement de la toxoplasmose encéphalitique.

**15.** Utilisation de:

a) un agent thérapeutique ou d'imagerie de diagnostic destiné à être administré par voie sanguine au cerveau;
b) un agoniste de type bradykinine vis-à-vis de la perméabilité de la barrière hémato-encéphalique ; et
c) un véhicule pharmaceutiquement acceptable pour la fabrication d'une composition pharmaceutique en vue d'une co-administration par voie intraveineuse à l'homme dans le but d'accroître la perméabilité de la barrière hémato-encéphalique audit agent.

FIG. 1

FIG. 2

FIG. 3

FIG. 4a

FIG. 4b

FIG. 5

FIG. 6

FIG. 7

FIG. 8

FIG. 9

FIG. 10

FIG.11

FIG. 12

FIG. 13

FIG. 14

FIG. 15